# EUROPEAN PATENT SPECIFICATION

(11) **EP 0 764 434 B1**
(45) Date of publication and mention of the grant of the patent: **28.03.2001**
(21) Application number: 95114726.3
(22) Date of filing: 19.09.1995
(51) Int. Cl.: A61F 13/15

(54) **Disposable absorbent article with pull out wings fixed in a delivery position**
Saugfähiger Einwegartikel mit herausziehbaren Laschen, die in einer Verpackungslage befestigt sind
Article absorbant jettable avec des rabats à tirer fixés dans une position de délivrance

(43) Date of publication of application: 26.03.1997
(73) Proprietor: THE PROCTER & GAMBLE COMPANY, Cincinnati, Ohio 45202 (US)
(72) Inventor: Schmitt, Achim, D-55424 Münster-Sarmsheim (DE)
(74) Representative: Hirsch, Uwe Thomas

(56) References cited:
- EP-A- 0 590 675
- WO-A-94/00091
- FR-A- 2 681 242

## Description

### FIELD OF THE INVENTION

The present invention relates to disposable absorbent articles such as sanitary napkins, pantiliners and incontinence pads that have side wrapping elements, also called wings for folding around the side edges of the wearer's undergarment on each longitudinal side edge of the article. The components are provided in a delivery position in which each of the side wrapping elements is folded upwards onto the wearer facing surface of the absorbent article and back onto itself. In this position the side wrapping elements can be unfolded by pulling the outermost edge of each side wrapping element in a transverse direction. The side wrapping elements are maintained in the delivery position by a separable delivery attachment means, preferably provided by crimping, between the wearer facing side of the side wrapping elements and the absorbent article.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perennial area of the body. Sanitary napkins in particular with side wrapping elements, often also referred to as side flaps or wings, are disclosed in the literature and are available in the marketplace.

Generally when sanitary napkins are provided with side flaps, the side flaps extend laterally from a central absorbent means and are intended to be folded around the edges of the wearer's panties in the crotch region. Commonly, the side flaps are provided with an attachment means for either affixing them to the underside of the wearer's panties or to the opposing side flap. The side flaps are particularly effective for preventing exudates from soiling the edges of the wearer's panties.

Sanitary napkins having wings or side flaps of various types are disclosed in U.S. Patent 4,687,478, entitled "Shaped Sanitary Napkin With Flaps", U.S. Patent 4,608,047, entitled "Sanitary Napkin Attachment Means", U.S. Patent 4,589,876, entitled "Sanitary Napkin" and its Reexamination Patent No. B1 4,589,876, U.S. Patent 4,285,343, entitled "Sanitary Napkin". Sanitary napkins having wings are commonly viewed as providing good protection against soiling. Typically the side flaps are provided on the sanitary napkins by either being attached to the garment facing surface of the absorbent article or by integrally extending from the lateral side edges of the absorbent article.

The side flaps are also typically folded inwards below or above the absorbent article for delivery such that a packaging can be provided to accommodate the basic width of the article without the side flaps. Tucked side flaps below the backsheet of the article which address this situation are known from PCT application PCT/US 9404926, PCT/US 9404927 and PCT/US 9404943. Alternative particular preferred embodiments of tucked side wrapping elements are disclosed in copending European application entitled "Disposable absorbent article having pull out wings", of the inventors J.A. Plumley, R. Geilich and M. Divo assigned to The Procter & Gamble Company, Cincinnati, Ohio, USA. In order to unfold the side flaps from this delivery position the user has to grab the edge of each side flap and unfold it into the basic use position in order to place the absorbent article into the undergarment and fold the wings around the side edges of the crotch region of the undergarment.

In WO 9 400 091 sanitary napkins with tucked flaps are disclosed which are held in a delivery position on the backsheet side of the napkins.

The delivery position of the side flaps typically is maintained during production, packaging and directly prior to unfolding the side wrapping elements. Maintaining this position is important since randomly unfolding side wrapping elements would cause production problems, e.g. the side wrapping elements could be caught by machine parts and ripped off; packaging problems, e.g. when a side wrapping element unintentionally unfolds and extends outside the package prior to closure of the package then the sealing of the package could be defect; and finally when the side flaps are unintentionally unfolded they may attach already prior to use to surfaces to which this is not intended.

Conventionally when the side wrapping elements are folded to the wearer or garment facing side of the absorbent article they are attached to each other or to the absorbent article by adhesive means and/or an additional piece of material such as a release paper. In this configuration the adhesive area on the garment facing surface of each of the side wrapping elements is covered by a single release paper which provides protection to the adhesive area and maintains the delivery position of the side wrapping elements. Typically all conventional means of maintaining the delivery position either requires additional materials such as adhesive or release papers or the conventional means are incompatible with novel tucked flap designs.

The present invention addresses the inconvenience caused by the various available versions of means to maintain the delivery position of side flaps of absorbent articles as well as the cost, material consumption and other concerns associated with release strips and adhesives in disposable absorbent articles.

Thus an objective of the present invention is to provide an absorbent article such as a sanitary napkin which is provided with an alternative to conventional means for maintaining the delivery position of side flaps. It is another objective of the present invention to provide an absorbent article with a pull out side flaps in a delivery position which does not require the use of a release strip for an attachment means on the garment facing side of the side flaps of the absorbent article to maintain the side flaps in the delivery position.

These and other objectives of the present invention will be more readily apparent when considered in reference with the following description and when taken in conjunction with accompanying drawings.

### SUMMARY OF THE INVENTION

The present invention relates to a disposable absorbent article such as a sanitary napkin, catamenial, pantiliner or incontinence pad, for wearing in an undergarment. The article has a longitudinal and a transverse axis. The article comprises a main body portion and side flaps which can be folded around the side edge of the undergarment to assume a protective position. The side flaps extend beyond the longitudinal side edge of the main body portion. The side flaps and the main body portion have a garment facing surface and a wearer facing surface. Typically the side flap has an optional attachment means for securing the side flap in the protective position. The article is provided prior to use with the side flap in a delivery position in which the side flap is folded onto the wearer facing surface of the article and the garment facing surface of the side flap is folded back onto itself such that the optional attachment means remains inaccessible prior to use of the side flap and to protect it from contamination.

According to the present invention the side flaps are maintained in the delivery position by a delivery attachment means which separates substantially without destruction of the materials of the side flaps upon pulling the side flaps parallel to the transverse axis but in opposite directions in order to unfold the tucked side flaps. The delivery attachment means joins in a separable way the wearer facing surface of each side flap to the wearer facing surface of the main body portion.

In another preferred embodiment according to the present invention each side flap has a laterally distal edge, a proximal edge and a bending axis between the distal and the proximal edges. Particularly preferred are embodiments in which the proximal edge of the side flaps are joined to the main body portion along the lateral outside edge of the main body portion of the absorbent article. In this configuration the side flaps are folded along the proximal edge, where they are joined to the main body portion, onto the wearer facing surface of the article and each side flap is folded along the bending axis back onto itself. In a preferred embodiment the optional attachment means on the side flaps is adhesive and the garment facing surface of the side flap coming into direct contact with the attachment means comprises a release coating. In this configuration the side flaps are ready to be pulled apart by the user of the absorbent article to unfold the article from its delivery position into the unfolded position ready to apply the absorbent article to an undergarment.

It is particularly preferred to have the bending axis somewhat further away from the distal edge of the side flap than from the proximal edge of the side flap since in this embodiment the distal edge of the side flap will extend beyond the periphery of the main body portion of the absorbent article such that the side flap can easily be grabbed by a wearer and pulled apart according to the above description. For sanitary napkins it is most preferred that the side flaps are provided by an integral part of the main body portion such that either the liquid pervious topsheet of the absorbent article, or the typically liquid impervious, but often breathable backsheet of the absorbent article or both extend to form the side flaps according to the present invention.

Preferably the delivery attachment means is provided along a portion of the proximal edge of the side flaps, more preferably the delivery attachment means is provided along two portions of the proximal edge of each of the side flaps which two portions are longitudinally spaced apart. In a highly preferred configuration of the delivery attachment means the side flap has a distal portion between the distal edge and the bending axis of the side flaps and a proximal portion between the proximal edge and the bending axis of the side flaps. A delivery attachment means is provided to join the proximal portion to the distal portion of the side flaps in conjunction with or without the other delivery attachment means. The delivery attachment means can be provided in many different ways but crimping or soldering are most preferred.

### BRIEF DESCRIPTION OF THE DRAWINGS

Figure 1 shows a partially cut away perspective view from below of an unfolded embodiment of a sanitary napkin according to the present invention.

Figure 2 shows a cross section along line 2 - 2 of the embodiment of the sanitary napkin of figure 1, however, in a delivery position.

Figure 3 shows a principle top plan view of a sanitary napkin in its delivery position with crimped delivery attachment means according to the present invention.

### DETAILED DESCRIPTION OF THE INVENTION

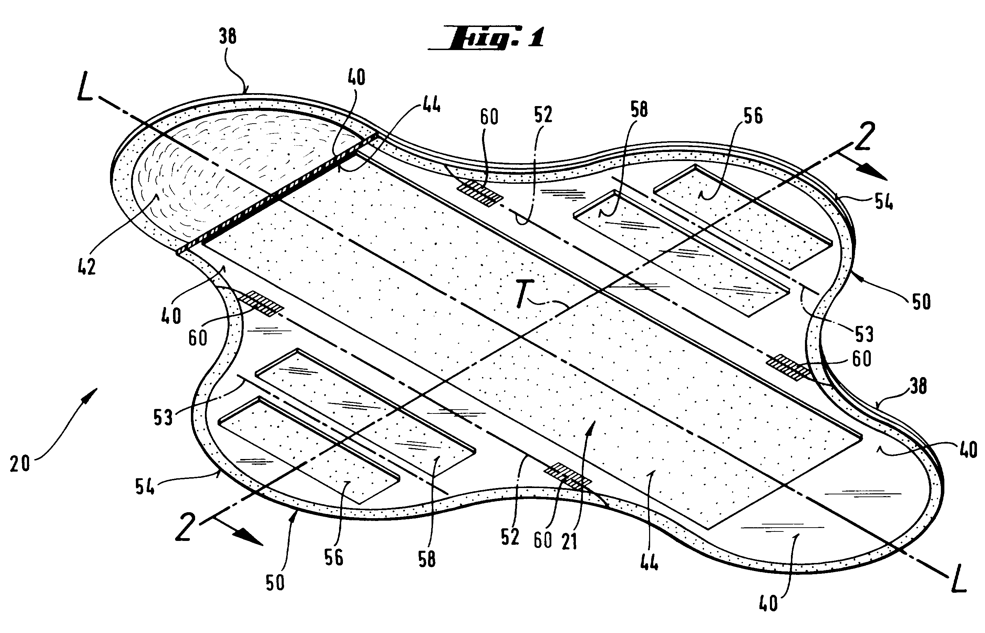

FIGS. 1-2 show one preferred embodiment of a disposable absorbent article of the present invention 20. The present invention relates to absorbent articles, such as sanitary napkins, panty liners and incontinence pads and will be explained in the following by reference to sanitary napkins. According to the present invention sanitary napkins have a main body portion 21 and a pair of side wrapping elements 50 which wrap the sides of the wearer's panties when the wearer places the sanitary napkin in her panties.

The sanitary napkin 20 has two surfaces, a liquid pervious wearer facing surface and a liquid impervious garment facing surface. The sanitary napkin 20 is shown in FIG. 1 as viewed from its garment facing surface. The garment facing surface of the sanitary napkin 20 is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

FIG. 1 shows the main body portion 21 of the sanitary napkin 20. The main body portion 21 comprises the portion of the sanitary napkin without the side wrapping elements 50. The main body portion 21 has two spaced apart longitudinal edges. A detailed description of a sanitary napkin and its main body portion is contained in U.S. Patent 4,690,680.

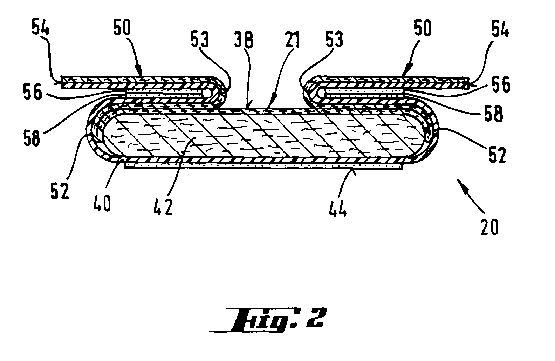

The main body portion 21 of the sanitary napkin 20 can be of any thickness, including relatively thick, relatively thin, or even very thin. The embodiment of the sanitary napkin 20 shown in Figures 1 and 2 is intended to be an example of a relatively thin sanitary napkin, preferably an "ultra-thin" sanitary napkin. An "ultra-thin" sanitary napkin 20 as described in U.S. Patents 4,950,264 and 5,009,653 preferably has a caliper of less than about 3 millimeters.

FIG. 1 or 2 show the individual components of the main body portion 21 of the sanitary napkin 20 of the present invention. The main body portion 21 of the sanitary napkin generally comprises at least three primary components. These include a liquid pervious topsheet 38, a liquid impervious backsheet 40, and an absorbent structure or core 42 positioned between the topsheet 38 and the backsheet 40. There are also occasions, however, when one or preferably more of these components, such as the backsheet and topsheet, can be replaced by a component that serves as part of the side wrapping elements 50 described below. The topsheet, the backsheet, and the absorbent core may be assembled in a variety of configurations known in the art (including so called "sandwich" products and "tube" products).

Several preferred sanitary napkin configurations are described generally in U.S. Patent 4,321,924, "Bordered Disposable Absorbent Article"; U.S. Patent 4,425,130, "Compound Sanitary Napkin"; U.S. Patent 4,950,264, "Thin, Flexible Sanitary Napkin"; U.S. Patent 5,308,346, "Elasticised Sanitary Napkin"; U.S. Patent Application Serial No. 08/096,121 entitled "Absorbent Articles Having Panty Covering Components That Naturally Wrap the Sides of Panties" filed July 22, 1993; and U.S. Patent Application Serial No. 08/124,180 entitled "Absorbent Articles Having Panty Covering Components Comprising Extensible Web Materials Which Exhibit Elastic-Like Behavior" filed September 17, 1993. The main body portion 21 of the sanitary napkin may also be comprised of one or more extensible components such as those sanitary napkins, and the like described in PCT Publication Nos. WO 93/01785 and 93/01786.

In general the three primary components of the main body portion need to satisfy the following:

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are typically selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the wearer remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the wearer facing surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps, side wrapping elements or wings.

When referring to the topsheet a multi layer structure or a mono layer structure is contemplated. The hybrid topsheet mentioned above is such a multi layer design but other multi layer topsheets such as primary and secondary topsheet designs are also considered.

The absorbent structure can include the following components: (a) optionally a primary fluid distribution layer preferably together with a secondary optional fluid distribution layer; (b) a fluid storage layer; (c) optionally a fibrous ("dusting") layer underlying the storage layer; and (d) other optional components.

### a Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared form polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins/panty liners.

An embodiment of the absorbent structure made according to the present invention comprises a double layer tissue laminate formed by folding the tissue onto itself. These layers can be joined to each other. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent structure according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent structure. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent structure according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings as shown in Figure 2.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-1401 and by Ethyl Corporation, Visqueen Division, of Terre Haute, Indiana, under the designation XP-39385. The backsheet is preferably embossed and/or matte finished to provide a more clothlike appearance. Further,. the backsheet can permit vapours to escape from the absorbent structure, i.e. be breathable, while still preventing exudates from passing through the backsheet. Also breathable backsheets comprising several layers, e.g. film plus non-woven structures, can be used and are preferably joined to each other.

### Side flaps or side wrapping elements

Figures 1-2 show a preferred embodiment of the sanitary napkin 20 assembled in a sandwich construction in which the topsheet 38 and the backsheet 40 have length and width dimensions generally larger than those of the absorbent core 42. The sanitary napkin 20 of the present invention comprises a pair of side wrapping elements 50 that extend laterally outward beyond the longitudinal side edges of the main body portion 21. In order to maintain the absorbent core in it's position and to provide structural integrity to the main body portion the topsheet is joined to the backsheet in any usual fashion for example adhesive gluing, welding, soldering or crimping the topsheet to the backsheet.

The side wrapping elements 50 can be of any suitable size and shape. The side wrapping elements 50 of the present invention may have any of the dimensions and characteristics set forth for the undergarment covering components in the aforementioned publication.

The side wrapping elements 50 can, as shown in FIGS. 1-2, be integral with the main body portion 21. Alternatively the side wrapping elements 50 can, however, be joined to the main body portion 21 in any suitable manner. The term "joined", generally as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element. Thus, the side wrapping elements 50 can be integral extensions of the topsheet 38 and backsheet 40 of the main body portion 21 as shown in Fig. 1 and 2. In other embodiments, instead of being integral, the side wrapping elements 50 can comprise a single or more separate component which are attached to the garment facing side of the absorbent article or which can be joined along the proximal edge 52 of the side wrapping elements 50 to the longitudinal side edge of the main body portion of the article 20.

Typically the side wrapping elements 50 comprise attachment means 56. The attachment means are typically paired by a release surface 58. As can be seen in figure 2 the side wrapping elements 50 are folded onto the wearer surface of the main body portion 21 of the absorbent article 20 and back onto itself along bending axis 53. In the preferred embodiment shown in the figures an attachment means 56 is provided on each side wrapping element 50. Also each attachment 56 is paired with a release surface 58 which preferably has a slightly larger area than the attachment means 56. In the delivery position shown in figure 2 of the article 20 the attachment means 56 rests on the release surface 58 so as to protect the attachment means 56 from any possible contamination prior to use of the absorbent article 20.

The attachment means 56 can be any attachment means typically used in the art. This includes such attachments as the male part of mechanical fasteners, so called hooks, which engage into the fibrous surface of the undergarment as the complementary female loop part. More typical the attachment means 56 is a pressure sensitive adhesive well known in the art which releasably adheres to the surface of the undergarment when placed in the use position. Suitable adhesives are Savare LA203 and LA303 of the Savare I.C. company of Milan in Italy, Coramelt 867 by Koemmerling in Pirmasens in Germany and Fuller H-2238ZP manufactured by the H.B. Fuller Co of Lueneburg in Germany. Suitable adhesive fasteners are also described in U.S. Patent 4,917,697.

The release surface 58 naturally has to be selected in accordance with the selection of the attachment means 56. For an adhesive attachment means a silicon coated release strip 58 or more preferably a coating of the release compound onto the garment facing surface of the side flap 50 in the region of the release surface 58, for example with silicon, has been found useful. In a similar fashion it is possible to use an adhesive coating onto the garment facing surface of the side flap to provide the attachment means 56 rather than the use of an adhesive tape which comprises a backing material with adhesive on either side in order to join it to the garment facing surface of the side flap 50 and provide the adhesive attachment means 56.

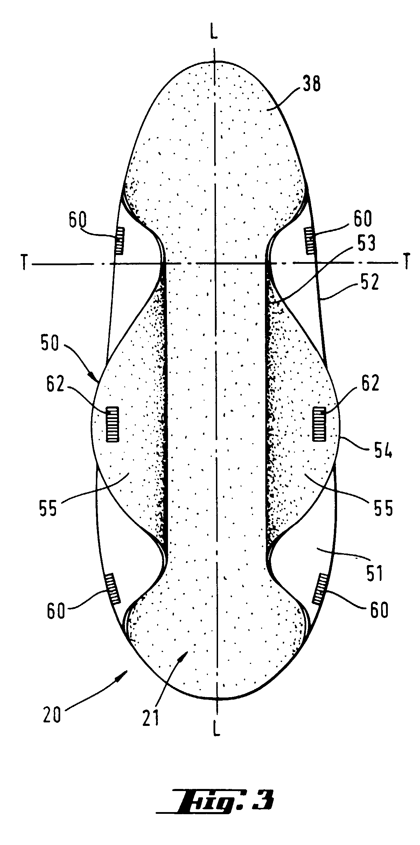

Also in figure 2 and 3 it can be seen that the distal edge 54 of the side flap 50 is further away from the bending axis 53 than the proximal edge 52 where the side flap is joined to the main body portion 21 of the absorbent article 20. By means of this difference in distance the distal edge 54 in the delivery position of the absorbent article as shown in figure 2 or 3 extends beyond the periphery of the main body portion 21 of the absorbent article such that grabbing the distal edge 54 of the side flap 50 in order to pull out the side flap 50 into a flat stretched out position is simplified even further.

In figure 3 it can be seen that between the proximal edge 52 and the bending axis 53 a proximal portion 51 of the side flap 50 is defined. The distal edge 54 together with the bending axis 53 enclose the distal portion 55 of the side flap 50. The particular form of the proximal edge 52 will provide the sanitary napkin 20 with a desirable slope when the side flaps are employed around the side edges of an undergarment. In order to maintain the side flaps in the position as shown in Figure 2 or 3 prior to use of the absorbent article it has been found useful to provide one or several distribution attachment means 60, 62 for each side flap.

In principle the distribution attachment means can be provided by any usual joining means known in the art provided the separating of the distribution attachment means does cause substantially no destruction of the material of the side flaps when they are unfolded in their usual way. By "substantially without destruction" it is meant that the product is not damaged to an extend that it cannot be used anymore or in other words "substantially without destruction" means that the wearer of such products would unfold the side flaps and believe that the product can still be used in the designed way. Distribution attachment means which cannot satisfy this criteria are not included in the present invention. The most preferred distribution attachment means is provided by crimping or soldering of material.

A preferred distribution attachment means is provided between the wearer facing surface of the side flap and the wearer facing surface of the main body portion. Such distribution attachment means 60 are indicated in Figure 1 after unfolding of the side flaps 50. In particular if distribution attachment means are provided along the proximal edge 52 of the side flaps 50 the desired maintenance of the delivery position can be achieved. A preferred configuration of two distribution attachment means 60 at two longitudinal spaced portions of the proximal edge 52 of the side flap 50 is shown in figure 1 and 3. Figure 3 also shows an additionally preferred distribution attachment means 62 which provides a separable joining between the proximal portion 51 and the distal portion 55 of the side flap 50. This distribution attachment means 62 can be employed alone or preferably in combination with other distribution attachment means.

It is also envisaged that the distribution attachment means 62 joins three layers: the distal portion 55, the proximal portion 51 of the side flap 50 and at least the outer most material layer of the main body portion 21 which provides the wearer facing surface, typically the topsheet 38. In another alternative embodiment it is envisaged that only the distribution attachment means 62 would be sufficient to maintain the delivery position of the absorbent article.

It is readily understandable how tucked side flaps according to the prior art which are folded essentially below the backsheet of a disposable absorbent article can employ the distribution attachment means according to the present invention such that such embodiments are also subject to the present invention.

The side wrapping elements 50 can be made from any of the materials used in the construction of the main body portion 21 of the sanitary napkin. The side wrapping elements 50 in the embodiment shown in FIGS. 1-2 preferably comprise a laminate of two materials such as the topsheet and the backsheet material. Separately attached side wrapping elements can be made in the same fashion or from a different laminate, e.g. of a soft extensible coverstock material such as an extensible spunbond nonwoven web or a soft extensible formed film, an optional intermediate layer such as a three dimensional formed film, and a backing such as a polyethylene film backsheet material.

Suitable material for the side wrapping elements include those useful to provide the topsheet, the backsheet and optionally intermediate layers. In this context suitable nonwoven webs include a product known as Spunbond PE, which can be obtained from Polybond, Incorporated of Waynesboro, VA, USA and a product known as COROLIND PE, which can be obtained from Corovin GMBH of Germany.

As intermediate layer or as topsheet material a variation of a three dimensional formed film known as DRI-WEAVE can be used. DRI-WEAVE is used as a topsheet on sanitary napkins manufactured by the Procter & Gamble Company, Cincinnati, Ohio under U.S. Patents 4,342,314 and 4,463,045 . The three dimensional film for use in the side wrapping elements can be apertured or not apertured all the way through as in the case of DRI-WEAVE topsheet material. For integral side wrapping elements the DRI-WEAVE topsheet can be formed so that the apertures are closed off on the longitudinal side of the film which forms the wearer facing side of the side wrapping elements.

The backsheet material can be for example the backsheet material mentioned above. This polyethylene film can be joined to form the laminate of the side wrapping elements together with the nonwoven and/or the formed film by any suitable method such as by a spiral application of adhesives or slot coating, or it can be welded or extruded together.

The composite laminate preferably has a caliper of between 0.13 - 1.5 mm and is capable of extending between about 25-80% without tearing. The particular laminate is chosen to provide a soft body-contacting surface, good extensibility if extensibility is desired, good resistance to folding and crumpling and a liquid barrier.

The garment facing surface of the sanitary napkin 20 may include, and preferably does include, fasteners for attaching the sanitary napkin to the wearer's undergarment. Figure 1 and 2 shows the central pad fastener 44 which is adapted to secure the portion of the sanitary napkin underlying the main body portion 21 to the crotch region of an undergarment. Any types of fasteners known in the art can be used. Fasteners comprising adhesives have been found to work well for this purpose, with pressure-sensitive adhesives being preferred. Before the sanitary napkin 20 is placed in use, if an adhesive fastener is used, the adhesive is typically covered with a removable cover strip or release liner in order to keep the adhesive from sticking to a surface other than the crotch portion of the panty prior to use. Suitable release liners and adhesive fasteners are described in the U.S. Patent 4,917,697. Suitable adhesives are those which can also be used for adhesive attachment means 56.

In a preferred arrangement the adhesive 44 is provided by a pair of spaced apart longitudinally-oriented strips or zones of adhesive that are centered about the longitudinal centerline L. Also centered around the longitudinal centerline L are the attachment means of adhesive 56 on the side wrapping elements. They can be provided with the same or with an alternative adhesive material. The principle placement relative to bending axis 53 of the attachment means 56 and the release surface 58 can also be exchanged such that release surface 58 is laterally outside the attachment means 56. Also a single attachment means on one side flap 50 can be used particularly when attaching the side flaps in a position where they circle the crotch region of the undergarment.

The sanitary napkin 20 of the present invention e.g. can be used by removing the release liner from adhesive 44 and thereafter placing the sanitary napkin 20 in a panty so that the adhesive 44 contacts the panty and maintains the sanitary napkin in position within the panty during use. The side wrapping elements 50 are pulled out and wrapped around the sides of the wearer's panties. The side wrapping elements 50 then assume an in-use position and the attachment means 56 attaches the side wrapping elements 50 to the undergarment.

### Alternative Embodiments

Numerous alternative embodiments of the present invention are possible. For example, the side wrapping elements are preferably mirror images of each other, and are symmetrical about the longitudinal centerline. However, it should be understood that the shape and location of the side wrapping elements described herein are those of a preferred embodiment, and other embodiments are also possible. For example, while the side wrapping elements 50 are shown as extending symmetrical from each longitudinal edge of the main body portion, there may be one side wrapping element extending further than the other one. Further, the side wrapping elements 50 may be offset along the longitudinal centerline more towards one end edge of the main body portion than the other.

The terms "panty liner" refer to absorbent articles that are less bulky than sanitary napkins which are generally worn by women between their menstrual periods. Suitable absorbent articles in the form of pantiliners that can benefit from the present invention described herein are disclosed in U.S. Patent 4,738,676 entitled "Pantiliner".

The term "incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults, children or infants. Suitable incontinent articles that can be provided with the side wrapping elements described herein are disclosed in U.S. Patent 5,300,054 and U.S. Patent 5,304,161.

## Claims

1. Disposable absorbent article (20) for wearing in an undergarment, said article (20) having a longitudinal (L) and a transverse (T) axis, said article (20) comprising a main body portion (21), and side flaps (50) extending beyond the longitudinal side edge of said main body portion (21), which side flaps (50) can be folded around the side edge of said undergarment to assume a protective position, said main body portion (21) and side flaps (50) having a garment facing surface and a wearer facing surface,
said side flaps (50) being in a delivery position prior to use of said article (20) in which each said side flap (50) is folded onto said wearer facing surface of said main body portion (21) and said garment facing surface of each said side flap (50) is folded back onto itself such that said side flaps (50) can be pulled in the direction of said transverse axis (T) away from each other to unfold said side flaps (50), wherein said side flaps (50) are maintained in said delivery position by delivery attachment means (60), said delivery attachment means (60) separating substantially without destruction of the materials of said side flaps (50) upon said unfolding of said side flaps (50) and
wherein said delivery attachment means (60) are joining said wearer facing surface of each of said side flaps (50) to said wearer facing surface of said main body portion (21).

2. Article (20) according to claim 1 wherein said side flaps (50) are joined to said main body portion (21) on each laterally opposing side edge of said main body portion (21) and each said side flap (50) has a laterally distal edge (54), proximal edge (52) and a bending axis (53) between said distal (54) and proximal (52) edge, and said side flaps (50) are folded along said proximal edge (52) onto said wearer facing surface of said main body portion (21) and each of said flaps (50) is folded along said bending axis (53) back onto itself into said delivery position.

3. Article (20) according to claim 2 wherein said bending axis (53) is further away from said distal edge (54) of said side flap (50) than from said proximal edge (52) of said side flap (50).

4. Article (20) according to claim 2 or 3 wherein said delivery attachment means (60) are provided along a portion of said proximal edge (52) of said side flaps (50).

5. Article (20) according to claim 2 or 3 wherein said delivery attachment means (60) is provided along two longitudinally spaced apart portions of said proximal edge (52) of each of said side flaps (50).

6. Article (20) according to claim 2, 3, 4 or 5 wherein each side flap (50) has a distal portion (55) between said distal edge (54) and said bending axis (53) and a proximal portion (51) between said proximal edge (52) and said bending axis (53) and said article (20) is provided with a delivery attachment means (60) between said distal portion (55) and said proximal portion (51) of each of said side flaps (50).

7. Article (20) according to any of the preceding claims wherein said article (20) is a sanitary napkin which comprises a liquid pervious topsheet (38), a backsheet (40) and an absorbent core (42) placed between said topsheet (38) and said backsheet (40), said topsheet (38) being joined to said backsheet (40) and said side flaps (50) are integral with at least one of said topsheet (38) and said backsheet (40).

8. Article (20) according to claim 7 wherein an adhesive coating (56) is provided onto said garment facing surface of each said side flap (50) transversely outside of said bending axis (53) and a release coating (58) is provided onto said garment facing surface of each said side flap (50) transversely inside said side flap (50) in order to facilitate a fixation of said article (20) in said protective position.

9. Article (20) according to any of the preceding claims wherein said delivery attachment means (60) is provided by crimping.

## Patentansprüche

1. Absorbierender Einwegartikel (20) zum Tragen in einer Unterwäsche, wobei der Artikel (20) eine längs verlaufende (L) und eine quer verlaufende (T) Achse hat, wobei der Artikel (20) einen Hauptkörperbereich (21) und Seitenklappen (50), welche sich über den längs verlaufenden Seitenrand des Hauptbereichs (21) erstrecken, umfaßt, wobei die Seitenklappen (50) um den Seitenrand der Unterwäsche herum gefaltet werden können, um eine Schutzposition einzunehmen, wobei der Hauptkörperbereich (21) und die Seitenklappen (50) eine wäscheseitige Oberfläche und eine trägerseitige Oberfläche haben,
wobei sich die Seitenklappen (50) vor der Verwendung des Artikels (20) in einer Auslieferungsposition befinden, in welcher jede Seitenklappe (50) auf die trägerseitige Oberfläche des Hauptkörperbereichs (21) gefaltet ist und die wäscheseitige Oberfläche jeder Seitenklappe (50) zurück auf sich selbst gefaltet ist, derart, daß die Seitenklappen (50) in Richtung der quer verlaufenden Achse (T) weg voneinander gezogen werden können, um die Seitenklappen (50) zu entfalten, in welchem die Seitenklappen (50) durch Auslieferungs-Befestigungsmittel (60) in der Auslieferungsposition gehalten werden, wobei die Auslieferungs-Befestigungsmittel (60) im wesentlichen ohne Zerstörung der Materialien der Seitenklappen (50) beim Entfalten der Seitenklappen (50) trennen, und in welcher die Auslieferungs-Befestigungsmittel (60) die trägerseitige Oberfläche jeder Seitenklappe (50) mit der trägerseitigen Oberfläche des Hauptkörperbereichs (21) verbinden.

2. Artikel (20) nach Anspruch 1, in welchem die Seitenklappen (50) mit dem Hauptkörnerbereich (21) auf jedem sich seitlich gegenüber liegenden Seitenrand des Hauptkörperbereichs (21) verbunden sind und jede Seitenklappe (50) seitlich einen Distalrand (54), einen Proximalrand (52) und eine Biegeachse (53) zwischen dem distalen (54) und proximalen (52) Rand hat, und wobei in der Auslieferungsposition die Seitenklappen (50) entlang des Proximalrandes (52) auf die trägerseitige Oberfläche des Hauptkörperbereichs (21) gefaltet sind und jede der Klappen (50) entlang der Biegeachse (53) auf sich selbst gefaltet ist.

3. Artikel (20) nach Anspruch 2, in welchem die Biegeachse (53) weiter entfernt vom Distalrand (54) des Seitenklappe (50) ist als vom Proximalrand (53) der Seitenklappe (50).

4. Artikel (20) nach Anspruch 2 oder 3, in welchem die Auslieferungs-Befestigungsmittel (60) entlang einem Bereich des Proximalrandes (52) der Seitenklappen (50) vorgesehen sind.

5. Artikel (20) nach Anspruch 2 oder 3, in welchem die Auslieferungs-Befestigungsmittel (60) entlang zwei in Längsrichtung voneinander in Abstand liegender Bereiche des Proximalrandes (52) jeder der Seitenklappen (50) vorgesehen sind.

6. Artikel 820) nach Anspruch 2, 3, 4 oder 5, in welchem jede Seitenklappe (50) einen Distalbereich (55) zwischen dem distalen Rand (54) und der Biegeachse (53) und einen Proximalbereich (51) zwischen dem proximalen Rand (52) und der Biegeachse (53) aufweist und wobei der Artikel (20) mit einem Auslieferungs-Befestigungsmittel (60) zwischen dem Distalbereich (55) und dem Proximalbereich (51) jeder der Seitenklappen (50) versehen ist.

7. Artikel (20) nach einem der vorstehenden Ansprüche, in welchem der Artikel (20) eine Damenbinde ist, welche eine flüssigkeitsdurchlässige Oberschicht (38), eine Unterschicht (40) und einen zwischen der Oberschicht (38) und der Unterschicht (40) angeordneten absorbierenden Kern (42) umfaßt, wobei die Oberschicht (38) mit der Unterschicht (40) verbunden ist und die Seitenklappen (50) mit wenigstens der Oberschicht (38) oder der Unterschicht (40) einstückig sind.

8. Artikel (20) nach Anspruch 7, in welchem ein Haftmittelüberzug (56) auf der wäscheseitigen Oberfläche jeder Seitenklappe (50) quer außerhalb der Biegeachse (53) vorgesehen ist und ein Abziehüberzug (58) auf der wäscheseitigen Oberfläche jeder Seitenklappe (50) quer innerhalb der Seitenklappe (50) vorgesehen ist, um eine Befestigung des Artikels (20) in der Schutzposition zu erleichtern.

9. Artikel (20) nach einem der vorstehenden Ansprüche, in welchem das Auslieferungs-Befestigungsmittel (60) durch Crimpen bereitgestellt wird.

## Revendications

1. Article absorbant jetable (20) destiné à être porté dans un sous-vêtement, ledit article (20) présentant un axe longitudinal (L) et un axe transversal (T), ledit article (20) comprenant une partie de corps principal (21) et des rabats latéraux (50) s'étendant au-delà du bord latéral longitudinal de ladite partie de corps principal (21), lesquels rabats latéraux (50) peuvent être pliés autour du bord latéral dudit sous-vêtement afin de prendre une position de protection, ladite partie de corps principal (21) et lesdits rabats latéraux (50) ayant une surface faisant face au vêtement et une surface faisant face au porteur,
lesdits rabats latéraux (50) étant dans une position de livraison avant l'utilisation dudit article (20), dans laquelle chaque dit rabat latéral (50) est plié sur ladite surface faisant face au porteur de ladite partie de corps principal (21), et ladite surface faisant face au vêtement de chaque dit rabat latéral (50) est repliée sur elle-même de façon que lesdits rabats latéraux (50) puissent être tirés dans la direction dudit axe transversal (T) à distance l'un de l'autre afin de déplier lesdits rabats latéraux (50),
dans lequel lesdits rabats latéraux (50) sont maintenus dans ladite position de livraison par des moyens de fixation de livraison (60), lesdits moyens de fixation de livraison (60) se séparant sensiblement sans destruction des matériaux desdits rabats latéraux (50) lors dudit dépliage desdits rabats latéraux (50), et
dans lequel lesdits moyens de fixation de livraison (60) réunissent ladite surface faisant face au porteur de chacun desdits rabats latéraux (50) à ladite surface faisant face au porteur de ladite partie de corps principal (21).

2. Article (20) selon la revendication 1, dans lequel lesdits rabats latéraux (50) sont réunis à ladite partie de corps principal (21) sur chaque bord latéralement opposé de ladite partie de corps principal (21), et chaque dit rabat latéral (50) a un bord latéralement distal (54), un bord proximal (52) et un axe de flexion (53) entre ledit bord distal (54) et ledit bord proximal (52), et lesdits rabats latéraux (50) sont pliés le long dudit bord proximal (52) sur ladite surface faisant face au porteur de ladite partie de corps principal (21), et chacun desdits rabats (50) est replié le long dudit axe de flexion (53) sur lui-même dans ladite position de livraison.

3. Article (20) selon la revendication 2, dans lequel ledit axe de flexion (53) est plus éloigné dudit bord distal (54) dudit rabat latéral (50) que dudit bord proximal (52) dudit rabat latéral (50).

4. Article (20) selon la revendication 2 ou 3, dans lequel lesdits moyens de fixation de livraison (60) sont prévus le long d'une partie dudit bord proximal (52) desdits rabats latéraux (50).

5. Article (20) selon la revendication 2 ou 3, dans lequel lesdits moyens de fixation de livraison (60) sont prévus le long de deux parties longitudinalement espacées dudit bord proximal (52) de chacun desdits rabats latéraux (50).

6. Article (20) selon la revendication 2, 3, 4 ou 5, dans lequel chaque rabat latéral (50) a une partie distale (55) entre ledit bord distal (54) et ledit axe de flexion (53), et une partie proximale (51) entre ledit bord proximal (52) et ledit axe de flexion (53), et ledit article (20) est prévu avec un moyen de fixation de livraison (60) entre ladite partie distale (55) et ladite partie proximale (51) de chacun desdits rabats latéraux (50).

7. Article (20) selon l'une quelconque des revendications précédentes, dans lequel ledit article (20) est une serviette hygiénique qui comprend une feuille de dessus (38) perméable aux liquides, une feuille de fond (40) et une âme absorbante (42) placée entre ladite feuille de dessus (38) et ladite feuille de fond (40), ladite feuille de dessus (38) étant réunie à ladite feuille de fond (40), et lesdits rabats latéraux (50) sont solidaires d'au moins une desdites feuille de dessus (38) et feuille de fond (40).

8. Article (20) selon la revendication 7, dans lequel un revêtement adhésif (56) est prévu sur ladite surface faisant face au vêtement de chaque dit rabat latéral (50) transversalement à l'extérieur dudit axe de flexion (53), et un revêtement de dégagement (58) est prévu sur ladite surface faisant face au vêtement de chaque dit rabat latéral (50) transversalement à l'intérieur dudit rabat latéral (50), afin de faciliter une fixation dudit article (20) dans ladite position de protection.

9. Article (20) selon l'une quelconque des revendications précédentes, dans lequel ledit moyen de fixation de livraison (60) est réalisé par gaufrage.
